# EUROPEAN PATENT SPECIFICATION

(11) **EP 2 748 136 B1**
(45) Date of publication and mention of the grant of the patent: **09.05.2018**
(21) Application number: 12762714.9
(22) Date of filing: 21.08.2012
(51) Int. Cl.: C07C 29/156, C07C 31/08

(54) **METHODS AND APPARATUS FOR SULFUR MANAGEMENT IN CATALYTIC MIXED-ALCOHOL SYNTHESIS**
VERFAHREN UND VORRICHTUNG FÜR SCHWEFELMANAGEMENT BEI DER KATALYTISCHEN MISCHALKOHOLSYNTHESE
PROCÉDÉS ET APPAREILS SERVANT À GÉRER LE SOUFRE LORS D'UNE SYNTHÈSE CATALYTIQUE D'UN MÉLANGE D'ALCOOLS

(30) Priority: 22.08.2011 US 201161526258 P
(43) Date of publication of application: 02.07.2014
(73) Proprietor: Albemarle Corporation, Baton Rouge, LA 70801 (US)
(72) Inventor: STITES, Ronald, C., Brighton, CO 80601 (US); TIRMIZI, Shakeel, H., Matawan, NJ 07747 (US); KHARAS, Karl, Louisville, CO 80027 (US)
(74) Representative: Uexküll & Stolberg
(86) International application number: PCT/US2012/051712
(87) International publication number: WO 2013/028686

(56) References cited:
- WO-A2-2008/048364
- WO-A2-2011/097648
- US-A1- 2010 280 287
- CHRISTENSEN J M ET AL: "Effects of H2S and process conditions in the synthesis of mixed alcohols from syngas over alkali promoted cobalt-molybdenum sulfide", APPLIED CATALYSIS A: GENERAL, ELSEVIER SCIENCE, AMSTERDAM, NL, vol. 366, no. 1, 15 September 2009 (2009-09-15), pages 29-43, XP026467718, ISSN: 0926-860X, DOI: 10.1016/J.APCATA.2009.06.034 [retrieved on 2009-06-30]

## Description

### FIELD OF THE INVENTION

The present invention generally relates to the field of processes for the chemical conversion of synthesis gas to alcohols, such as ethanol, using sulfided metal catalysts.

### BACKGROUND OF THE INVENTION

Synthesis gas (or syngas), a mixture of hydrogen (H₂) and carbon monoxide (CO), is a platform intermediate in the chemical and biorefining industries. Syngas may be converted into alkanes, olefins, oxygenates, or alcohols. These chemicals can be blended into, or used directly as, diesel fuel, gasoline, and other liquid fuels. Syngas can also be directly combusted to produce heat and power.

Syngas can be produced, in principle, from virtually any material containing carbon. Carbonaceous materials commonly include fossil resources such as natural gas, petroleum, coal, and lignite; and renewable resources such as lignocellulosic biomass and various carbon-rich waste materials. It is preferable to utilize a renewable resource to produce syngas because of the rising economic, environmental, and social costs associated with fossil resources.

When ethanol is desired from syngas, sulfided metal catalysts are commonly employed, usually with one or more base promoters to increase the selectivity to ethanol. During commercial operation, a reduction in sulfur concentration can occur at the active catalyst surface, thereby causing a loss in ethanol selectivity. In view of this problem, methods are needed to mitigate loss of sulfur from sulfided metal catalysts during for mixed-alcohol synthesis. If sulfur addition is necessary, it is preferable to reduce or eliminate feeding toxic H₂S gas to the process, and instead introduce sulfur compounds in a liquid phase, improving safety and reducing energy costs.

Sulfided metal catalysts tend to produce significant quantities of methanol, This methanol may be recovered and sold, or it may be subjected to additional reactions with syngas to produce higher alcohols from the methanol.

One approach involves separating at least some of the methanol produced from a reactor exit stream, and recycling the methanol back to the reactor inlet. Theoretically, all of the methanol produced could be recycled so that there is no net production of methanol (commonly known as recycling the methanol to "extinction"). As a fuel, methanol has lower market value than ethanol; therefore, it is desirable to recycle some or all methanol produced, to ultimately produce more ethanol. On the other hand, the fate of the recycled methanol needs to be considered. That is, the carbon atoms of the recycled methanol should preferably end up in the desired products, such as ethanol.

U.S. Patent App. No. 12/769,850, which is commonly owned with the present application, describes experimental catalyst deactivation associated with conversion of metal sulfides to metal carbides. In an attempt to mimic this catalyst deactivation, in an accelerated manner, a test protocol was developed in which large quantities of methanol were included in syngas feeds to the catalyst. It was found that methanol can strip sulfur from the mixed-alcohol catalyst. Addition of H₂S resulted in durable catalyst performance under accelerated-aging conditions. US 2010/280287 A1 discloses a method for producing ethanol from syngas in the presence of a sulfided metal catalyst, wherein a sulfur compound is co-fed with the syngas to the alcohol-synthesis reactor.

In addition to the above-mentioned need to mitigate loss of sulfur from sulfided metal catalysts, it is further desired to maintain the reaction selectivity to ethanol over time, when methanol is being recycled. Thus, there is a commercial need for stable sulfided metal catalysts and/or methods to improve stability and lifetime of these catalysts.

### SUMMARY OF THE INVENTION AND THE FURTHER DISCLOSURE

The present invention provides a method of producing ethanol from syngas, the method comprising:
(a) feeding syngas to an alcohol-synthesis reactor that contains a sulfided metal catalyst, under suitable conditions for converting the syngas into an intermediate stream comprising methanol, ethanol, and one or more sulfur-containing compounds selected from di-tert.-butyl polysulfide (DBPS) and dimethyl disulfide (DMDS);
(b) sending at least part of the intermediate stream to a separation unit, whereby at least a portion of the methanol is separated from the ethanol to form a methanol recycle stream and an ethanol product stream, and wherein at least a portion of the sulfur-containing compounds are contained in the methanol recycle stream;
(c) recycling some or all of the methanol recycle stream back to the alcohol-synthesis reactor to add sulfur to, or reduce sulfur loss from, the sulfided metal catalyst; wherein the methanol recycle stream has a sulfur-atom concentration of at least 10 ppm S; and
(d) optionally introducing one or more additional sulfur compounds selected from di-tert.-butyl polysulfide (DBPS) and dimethyl disulfide (DMDS) into the alcohol-synthesis reactor and/or into the methanol recycle stream, to add sulfur to, or reduce sulfur loss from, the sulfided metal catalyst.

Suitable apparatus for carrying out these methods are described herein. In some embodiments, the sulfided metal catalyst is a base-promoted cobalt-molybdenum-sulfur catalyst. The separation unit may include one or more distillation columns. When distillation is utilized, it is preferred to use a distillation column that is adapted (engineered) for separation of both methanol and sulfur-containing compounds.

Preferably, the methanol recycle stream has a sulfur-atom concentration optimized for the extent of methanol recycle back to the alcohol-synthesis reactor. The methanol recycle stream has a sulfur-atom concentration of at least 10 ppm S, such as about 50-500 ppm S, or about 100-300 ppm S. In certain embodiments, the methanol recycle stream has a sulfur-atom concentration of less than 200 ppm S.

In some embodiments, recycling methanol with sulfur, and optionally introducing additional sulfur, retards or eliminates a mechanism by which ethanol selectivity is otherwise lost as a result of recycling the methanol. This mechanism may be, or include, partial conversion of the sulfided metal catalyst to metal carbides.

The present disclosure also relates to optimum, non-obvious hydrogen sulfide concentrations when H₂S is employed as a sulfiding agent; such embodiments do not form part of the presently claimed invention. In some variations, a method of producing ethanol from syngas comprises:
(a) feeding syngas to an alcohol-synthesis reactor that contains a sulfided metal catalyst, under suitable conditions for converting the syngas into an intermediate stream comprising methanol and ethanol;
(b) co-feeding hydrogen sulfide with the syngas to the alcohol-synthesis reactor, to add sulfur to, or reduce sulfur loss from, the sulfided metal catalyst;
(c) sending at least part of the intermediate stream to a separation unit, whereby at least a portion of the methanol is separated from the ethanol to form a methanol recycle stream and an ethanol product stream; and
(d) recycling some or all of the methanol recycle stream back to the alcohol-synthesis reactor,
wherein the hydrogen sulfide is fed in step (b) at a concentration between about 50 ppm H₂S and about 400 ppm H₂S.

In some embodiments, the hydrogen sulfide is fed in step (b) at a concentration between about 50-250 ppm H₂S, such as about 75-150 ppm H₂S, or about 90-130 ppm H₂S. Preferably, the hydrogen sulfide is fed in step (b) at a concentration optimized for the extent of the recycling of the methanol recycle stream back to the alcohol-synthesis reactor. In some embodiments, hydrogen sulfide is fed in step (b) at a concentration optimized for retarding or eliminating a mechanism (such as carbide formation) by which ethanol selectivity is otherwise lost as a result of recycling the methanol.

The present disclosure includes a method of producing ethanol from syngas which does not form part of the presently claimed invention, the method comprising:
(a) feeding syngas to an alcohol-synthesis reactor that contains a sulfided metal catalyst, under suitable conditions for converting the syngas into an intermediate stream comprising methanol and ethanol;
(b) co-feeding hydrogen sulfide with the syngas to the alcohol-synthesis reactor, to add sulfur to, or reduce sulfur loss from, the sulfided metal catalyst;
(c) sending at least part of the intermediate stream to a separation unit, whereby at least a portion of the methanol is separated from the ethanol to form a methanol recycle stream and an ethanol product stream; and
(d) recycling some or all of the methanol recycle stream back to the alcohol-synthesis reactor,
wherein the hydrogen sulfide is fed in step (b) at a concentration optimized for the specific extent of the recycling some or all of the methanol recycle stream back to the alcohol-synthesis reactor.

### BRIEF DESCRIPTION OF THE FIGURES

FIG. 1 is an exemplary block-flow diagram according to some variations of the invention.
FIG. 2 is a plot of ethanol productivity versus time according to Example 1 herein, relating to accelerated catalyst aging with high methanol recycle rates.
FIG. 3 is a plot of ethanol selectivity versus time according to Example 1 herein, relating to accelerated catalyst aging with high methanol recycle rates.
FIG. 4 is a plot of CO conversion versus time according to Example 1 herein, relating to accelerated catalyst aging with high methanol recycle rates.

### DETAILED DESCRIPTION OF EMBODIMENTS OF THE INVENTION

This description will enable one skilled in the art to make and use the invention, and it describes several embodiments, adaptations, variations, alternatives, and uses of the invention, including what is presently believed to be the best mode of carrying out the invention. As used in this specification and the appended claims, the singular forms "a," "an," and "the" include plural referents unless the context clearly indicates otherwise. Unless defined otherwise, all technical and scientific terms used herein have the same meaning as is commonly understood by one of ordinary skill in the art to which this invention belongs.

Unless indicated to the contrary, the numerical parameters set forth in the following specification and attached claims are approximations that may vary depending at least upon the specific analytical technique. Any numerical value inherently contains certain errors necessarily resulting from the standard deviation found in its respective testing measurements.

As used herein, reference to "mixed alcohols" means methanol plus one or more alcohols selected from ethanol, propanol, and butanol, including all known isomers. While preferred embodiments are described in relation to high selectivities to ethanol, the invention can also be practiced in a manner that gives high selectivities to propanol and/or butanol, or even higher alcohols if desired.

The present invention will now be described by reference to the following detailed description, which characterizes some preferred embodiments but is by no means limiting.

In order to operate a stable alcohol-synthesis process for a commercially reasonable amount of time, such as in excess of 1000 hours, the presence of a sulfiding agent in the feed, or in another stream into the reactor, is beneficial. A sulfiding agent is desired to operate for an extended period of time without formation of less active and less selective transition-metal carbides. Also, a sulfiding agent is beneficial to operate for an extended period of time without deterioration of ethanol selectivity or productivity.

Some variations of the invention are premised on the discovery that reduction of alcohol selectivity is related to the stripping of sulfur from sulfided metal catalysts. The metal sulfides, when sulfur loss occurs, can form metal carbides. Sulfur stripping may be caused by recycling methanol. It has been shown through experimentation that the addition of sulfur compounds surprisingly retards or eliminates the mechanism by which selectivity is lost as a result of recycling methanol.

It is possible to utilize gas-phase hydrogen sulfide (H₂S) as a sulfiding agent. H₂S may be introduced into a syngas feed stream and then fed to a mixed-alcohol reactor. Although it is generally known that H₂S may be included in feed streams for mixed-alcohol synthesis, the prior art does not teach preferred concentration ranges of H₂S from the standpoint of ethanol selectivity.

The co-inventors of the present application have found, by experimentation, that preferred H₂S concentrations are at least about 50 ppm (by volume) and less than about 400 ppm. Preferred H₂S concentrations, in various embodiments, include about 50, 60, 70, 80, 90, 100, 110, 120, 130, 140, and 150 ppm H₂S. In other embodiments, preferred H₂S concentrations include about 175, 200, 225, 250, 275, 300, 325, 350, 375, or 400 ppm H₂S.

There is not a single optimum H₂S concentration applicable to all conditions, as will be appreciated by a skilled artisan. Rather, the optimum H₂S concentration (to maximize ethanol selectivity or yield) will generally depend on (i) catalyst requirements, (ii) reactor conditions and, as has been unexpectedly discovered herein, (iii) the extent of methanol recycle.

As is well-known, H₂S is a dangerous gas, associated with transport, storage, and regulatory concerns. It would therefore be preferable to employ liquid-phase sulfur-containing compounds for sulfiding, instead of H₂S. Exemplary sulfur-containing compounds include, but are by no means limited to, methyl sulfide, dimethyl sulfide, dimethyl disulfide, di-tert-butyl disulfide, and analogues, derivatives, oligomers, polymers, reaction products, and combinations thereof.

It is known that sulfides and disulfides can form polymers with several sulfur atoms and sulfur-sulfur bonds. For example, di-tert-butyl disulfide may be present as, or characterized as, di-tert-butyl polysulfide, wherein additional sulfur atoms (such as 1, 2, 3, 4, or more additional S atoms) are contained between the di-tert-butyl groups. Di-tert-butyl polysulfide is a preferred sulfur compound in certain embodiments. One commercial source of di-tert-butyl polysulfide is SulfrZol® 54 (Lubrizol).

In various embodiments, sulfur may be introduced by injecting, in dissolved form or another effective form, one or more compounds selected from elemental sulfur, hydrogen sulfide, dimethyl sulfide, diethyl sulfide, dimethyl disulfide, any isomers of dibutyl polysulfide (such as di-tert-butyl polysulfide), any isomers of dioctyl polysulfide, diphenyl polysulfide, dicyclohexyl polysulfide, methylthiol, ethylthiol, cysteine, cystine, methionine, potassium disulfide, cesium disulfide, and/or sodium disulfide. Various isomers of these compounds may be used. For example, cysteine may be present as L-cysteine, D-cysteine, or D,L-cysteine mixtures.

According to the presently claimed invention, the intermediate stream as defined in step (a) of claim 1 comprises one or more sulfur-containing compounds selected from di-tert.-butyl polysulfide (DBPS) and dimethyl disulfide (DMDS).

For the purpose of adding fresh sulfur to the reactor, one or more of these sulfur-containing compounds may be dissolved in, for example, toluene or other organic solvents. For the disulfides of potassium, sodium, or cesium, effective solvents may be selected from alcohols, short-chain polyethylene glycols, acetonitrile, DMF, DMSO, or THF, for example.

Effective sulfiding compounds need to be able to deposit sulfur atoms, or sulfur-containing species, onto a surface of a mixed-alcohol catalyst. When a liquid sulfur compound is employed, preferably the performance of the catalyst is substantially similar to the catalyst performance when H₂S is the sulfiding agent. "Substantially similar" performance means a similar product distribution at similar carbon conversion.

Without being limited to any particular theory, it is believed that some sulfiding compounds are capable of converting (to some extent) to H₂S under reactor conditions. The *in situ* generation of H₂S then allows for deposition of sulfur on the catalyst surface, in the same or similar manner as if H₂S was fed directly to the reactor. The detailed mechanism may or may not involve molecular H₂S in the vapor phase. That is, adsorbed hydrogen sulfide or other surface species (such as HS⁻ or HS·) may be involved in the process to deposit sulfur onto the mixed-alcohol catalyst.

The reaction products of sulfur compounds may include not only H₂S, but also other light sulfur compounds, such as carbonyl sulfide (COS) and methanethiol (CH₃SH). The reaction products may form in the mixed-alcohol reactor or at any point downstream, including during distillation and recycle. In some embodiments, it is believed that the sulfur-containing reaction products are more effective for sulfiding than are the initial sulfur compounds. Without being limited by any hypotheses, reaction products that are lighter sulfur compounds may be more effective for chemical reasons (e.g., faster sulfiding kinetics) or for physical reasons (e.g., higher rates of mass transfer to the surface).

According to the present invention, one or more sulfur compounds (and/or their reaction products) are recycled from a mixed-alcohol product stream to the catalytic reactor. Distillation may be employed to remove a portion or all of the methanol and a portion or all of the sulfur-containing compounds from the mixed-alcohol stream for recycle. Other separation means may be employed, as will be appreciated. When the separation is based on volatility differences, it is preferred that the selected sulfur compound has a volatility similar to the volatility of methanol. An advantage of distillation is that the same distillation column(s) used for methanol removal and recycle may be used for removal and recycle of sulfur compounds.

In addition to the recycle of sulfur, light sulfur-containing compounds (such as dimethyl disulfide) may be added to the methanol recycle stream. This addition of sulfur can maintain the sulfur inventory needed to preserve catalyst selectivity, in some embodiments. The amount of fresh sulfur relative to recycled sulfur can vary, depending on catalyst sulfur requirements, process conditions, process upsets, control methodologies, and so on. In preferred embodiments, the amount of fresh sulfur needed is reduced by recycling sulfur compounds from the product stream.

When sulfur compounds other than H₂S are employed, the optimum concentration will generally depend on (i) catalyst requirements, (ii) reactor conditions and, as has been discovered herein, (iii) the extent of methanol recycle. Preferred concentrations of sulfur compounds, on a S-atom basis, include about 50 ppm S, 75 ppm S, 100 ppm S, 125 ppm S, 150 ppm S, 175 ppm S, 200 ppm S, or more.

In some embodiments, a selected sulfur compound may be relatively inert, but high recycle ratios can be employed so that the sulfur compound (and/or its reaction products) reaches a sufficient steady-state concentration for effective sulfiding. In some of these particular embodiments, the selected sulfur compound is dimethyl sulfide.

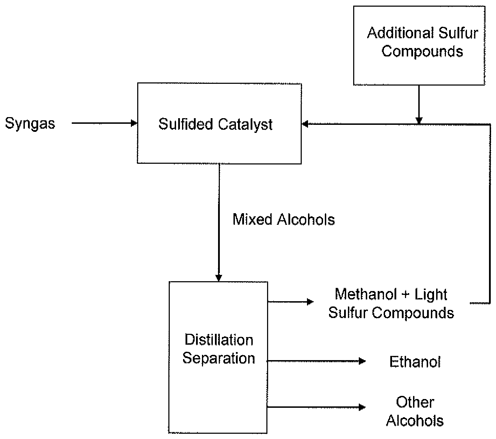

FIG. 1 is an exemplary block-flow diagram illustrating some variations of the invention. In FIG. 1, syngas passes over a sulfided catalyst in a reactor to produce mixed alcohols, which are then fed to a distillation unit. A stream that includes methanol and sulfur compounds is recovered, such as in the distillation overhead or in a sidedraw. This stream is recycled back to the reactor, optionally with additional sulfur introduced to the recycle stream or directly to the reactor.

When distillation is employed, as in FIG. 1, it may include one or more columns, depending on the desired overall separation and cost factors. Columns may be designed with any known distillation column configuration, including packed columns, bubble-cap trays, sieve trays, and so on. A skilled artisan can carry out process simulations to predict separations and estimate the number of equilibrium and actual stages necessary.

Process simulations can also predict the splits of sulfur-containing compounds. It has been found, based on simulations, that several light sulfur compounds preferentially split with methanol in a mixed-alcohol separation.

The process of FIG. 1 allows for a great deal of flexibility in how much sulfur is returned to the reactor. Additional sulfur may be introduced with the recycle stream, if sulfur recovery in distillation is insufficient or there are other sulfur losses. On the other hand, if it is not desired to recycle all of the sulfur, then a portion of the methanol stream is not returned to the reactor. This embodiment may be useful, for example, at start-up or during other transient operations in which the sulfur demand may be less. In certain embodiments, all of the sulfur contained in the methanol stream is recycled but no additional sulfur is introduced. In some embodiments, the quantity of sulfur being recycled with methanol is sufficient to overcome any accelerated sulfur stripping of the catalyst due to methanol.

There are several direct ways to analyze the level of catalyst sulfiding and then adjust the quantity of sulfur returning to the reactor. In some embodiments, the sulfur content may be measured in the reactor effluent, or in one or more distillation outlet streams. The sulfur content may be measured in the product of interest, e.g. ethanol.

There are also several indirect means for analyzing the catalyst sulfiding, based on process performance. In some embodiments, selectivity or productivity to ethanol (or another product) is dynamically measured and, based on those measurements, more or less sulfur is introduced. Preferably, recycling the methanol with sulfur can preserve catalyst selectivity to ethanol. In some embodiments, measurements are based on the premise that if the catalyst is insufficiently sulfided, carbides may form and adjust carbon selectivity towards methane. Reduction of CO conversion would also be expected as a result of sulfide to carbide conversion.

In some embodiments with Co/Mo/S catalysts, sulfur is recycled (optionally with additional sulfur injected) to control the molar ratio S:Co to between about 1.2 to about 2 or higher, up to about 4. In addition to the techniques described above, catalyst samples may be occasionally analyzed to measure S:Co and, if needed, additional sulfur may be introduced. Alternately, experiments may be separately conducted to establish that additional sulfur is necessary at certain times, or as a continuous injection in prescribed amounts, or some other program, in order to control (maintain) the S:Co ratio.

During the conversion of syngas to alcohols over mixed-alcohol catalysts, the mechanism for chain growth may involve organic acids as intermediates. A possible mechanism for chain growth is the insertion of CO into the C-O bond of an alcohol. Without being limited by any particular hypothesis, it is believed that under certain conditions an adsorbed acid is reduced to the corresponding normal alcohol, which may progress via the base-catalyzed reduction of C=O bonds by sulfides. A reducing sulfided catalyst may be involved either directly or indirectly, The metals may react directly in their reduced state or they may release sulfur to accomplish the reduction. Upon reduction, a C=O group is replaced by a CH₂ group.

Any sulfided mixed-alcohol catalyst may be employed in this invention. In some embodiments, a mixed-alcohol sulfided catalyst comprises cobalt, molybdenum, and sulfur. Some embodiments use one or more catalyst compositions described in U.S. Patent. No. 7,923,405, issued April 12, 2011 or U.S. Patent App. No. 12/769,850, filed April 29, 2010.

The sulfided mixed-alcohol catalyst may be base-promoted. Base promoters can enhance the production of alcohols from syngas. By "base promoter" it is meant one or more metals that promote the production of alcohols. Base promoters may be present in free or combined form. The base promoter may be present as a metal, oxide, carbonate, hydroxide, sulfide, as a salt, in a compound with another component, or some combination of the above.

The catalyst may take the form of a powder, pellets, granules, beads, extrudates, and so on. Some embodiments benefit from small particle sizes (higher surface area) in the bulk catalyst. Some embodiments benefit from the presence of relatively large pores or channels in the bulk catalyst. In some embodiments, the catalyst particles are present in a slurry or other homogeneous phase.

When a catalyst support is optionally employed, the support may assume any physical form such as pellets, spheres, monolithic channels, films, etc. The supports may be coprecipitated with active metal species; or the support may be treated with the catalytic metal species and then used as is or formed into the aforementioned shapes; or the support may be formed into the aforementioned shapes and then treated with the catalytic species. In embodiments of the invention that employ a catalyst support, the support is preferably (but not necessarily) a carbon-rich material with large mesopore volume, and further is preferably highly attrition-resistant.

In some embodiments, conditions effective for producing alcohols from syngas include a feed hydrogen/carbon monoxide molar ratio (H₂/CO) from about 0.2-4.0, preferably about 0.5-2.0, and more preferably about 0.5-1.5. These ratios are indicative of certain embodiments and are not limiting. It is possible to operate at feed H₂/CO ratios less than 0.2 as well as greater than 4, including 5, 10, or even higher. It is well-known that high H₂/CO ratios can be obtained with extensive steam reforming and/or water-gas shift in operations prior to the syngas-to-alcohol reactor.

In embodiments wherein H₂/CO ratios close to 1:1 are desired for alcohol synthesis, partial oxidation of the carbonaceous feedstock may be utilized. In the absence of other reactions, partial oxidation tends to produce H₂/CO ratios close to unity, depending on the composition of the feedstock.

When, as in certain embodiments, relatively low H₂/CO ratios are desired, the reverse water-gas shift reaction (H₂ + CO₂ → H₂O + CO) may be utilized to consume hydrogen and thus lower H₂/CO. In some embodiments, CO₂ produced during alcohol synthesis or elsewhere, can be recycled to the reformer to decrease the H₂/CO ratio entering the alcohol-synthesis reactor. Other chemistry and separation approaches may be taken to adjust the H₂/CO ratios prior to converting syngas to alcohols, as will be appreciated. For example, certain commercial membrane systems are known to be capable of selectively separating H₂ from syngas, thereby lowering the H₂/CO ratio.

In some embodiments, conditions effective for producing alcohols from syngas include reactor temperatures from about 200-400°C, preferably about 250-350°C; and reactor pressures from about 20-507 bar (20-500 atm), preferably about 51-203 bar (50-200 atm) or higher. Generally, productivity increases with increasing reactor pressure. Temperatures and pressures outside of these ranges may be employed. In some embodiments, conditions effective for producing alcohols from syngas include average reactor residence times from about 0.1-10 seconds, preferably about 0.5-2 seconds.

In general, the specific selection of catalyst configuration (geometry), H₂/CO ratio, temperature, pressure, residence time (or feed rate), and other reactor-engineering parameters will be selected to provide an economical process. These parameters are not regarded as critical to the present invention. It is within the skill of a person skilled in the art, having read the present disclosure, to experiment with different reactor configurations and conditions to optimize selectivity to ethanol or another product.

In some preferred embodiments, the ethanol selectivity is higher, preferably substantially higher, than the methanol selectivity. The product stream from the reactor may include C₃₊ alcohols, as well as non-alcohol oxygenates such as aldehydes, esters, carboxylic acids, and ketones. These other oxygenates may include, for example, acetone, 2-butanone, methyl acetate, ethyl acetate, methyl formate, ethyl formate, acetic acid, propanoic acid, and butyric acid.

In this example, a Co/Mo/S/K mixed-alcohol catalyst is evaluated for over 100 hr, including about 50 hr accelerated aging at the end of the run. Sulfiding agents tested include H₂S at 400 ppm and 110 ppm, DBPS at 175 ppm S equivalent, and DMDS at 108 ppm S equivalent.

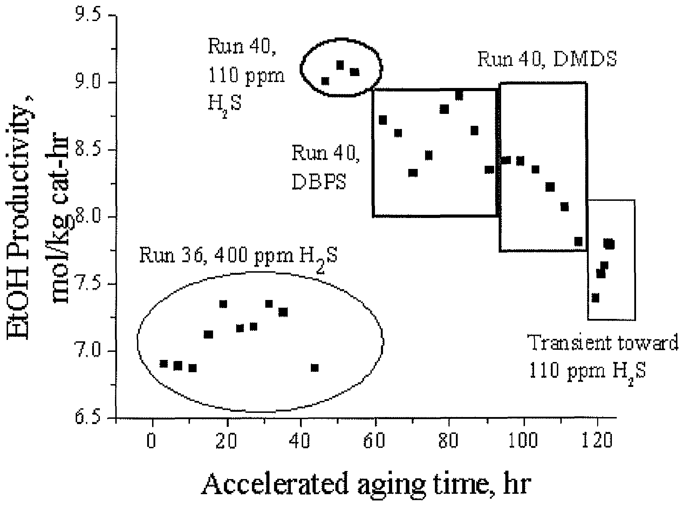

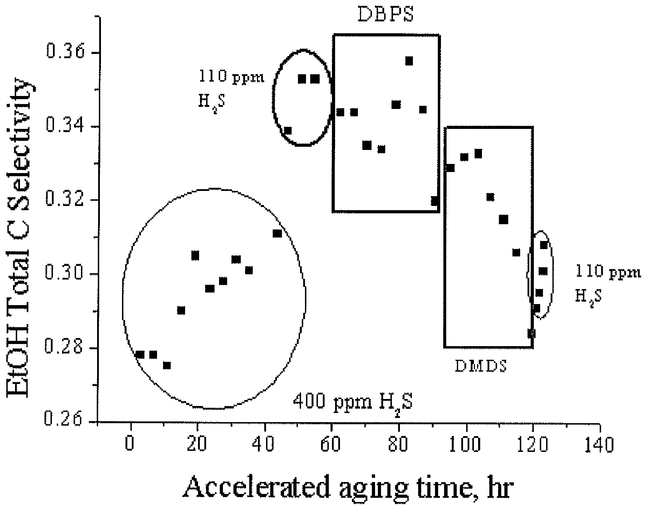

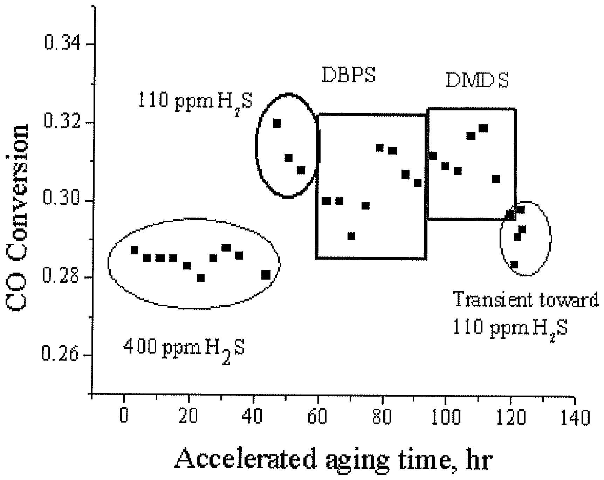

Experimental data are shown in FIGS. 2-4. One observation is that 400 ppm H₂S is inferior to 110 ppm H₂S, for ethanol productivity (FIG. 2), ethanol selectivity (FIG. 3), and CO conversion (FIG. 4). The performance is comparable with either DBPS or DMDS initially. There appears to be a reduction in ethanol productivity and selectivity with DMDS as a function of accelerated aging time. The reason may be due to DMDS being introduced at 108 ppm S, compared to 175 ppm S for DBPS.

In this detailed description, reference has been made to multiple embodiments of the invention and non-limiting examples relating to how the invention can be understood and practiced. Other embodiments that do not provide all of the features and advantages set forth herein may be utilized, without departing from the scope of the invention. This invention incorporates routine experimentation and optimization of the methods described herein. Such modifications and variations are considered to be within the scope of the invention defined by the appended claims.

Where methods and steps described above indicate certain events occurring in certain order, those of ordinary skill in the art will recognize that the ordering of certain steps may be modified and that such modifications are in accordance with the variations of the invention. Additionally, the steps may be performed concurrently in a parallel process when possible, as well as performed sequentially.

The invention shall only be limited by what is claimed.

## Claims

1. A method of producing ethanol from syngas, said method comprising:
(a) feeding syngas to an alcohol-synthesis reactor that contains a sulfided metal catalyst, under suitable conditions for converting said syngas into an intermediate stream comprising methanol, ethanol, and one or more sulfur-containing compounds selected from di-tert.-butyl polysulfide (DBPS) and dimethyl disulfide (DMDS);
(b) sending at least part of said intermediate stream to a separation unit, whereby at least a portion of said methanol is separated from said ethanol to form a methanol recycle stream and an ethanol product stream, and wherein at least a portion of said sulfur-containing compounds are contained in said methanol recycle stream;
(c) recycling some or all of said methanol recycle stream back to said alcohol-synthesis reactor to add sulfur to, or reduce sulfur loss from, said sulfided metal catalyst; wherein said methanol recycle stream has a sulfur-atom concentration of at least 10 ppm S; and
(d) optionally introducing one or more additional sulfur compounds selected from di-tert.-butyl polysulfide (DBPS) and dimethyl disulfide (DMDS) into said alcohol-synthesis reactor and/or into said methanol recycle stream, to add sulfur to, or reduce sulfur loss from, said sulfided metal catalyst.

2. The method of claim 1, wherein said sulfided metal catalyst is a base-promoted cobalt-molybdenum-sulfur catalyst.

3. The method of claim 1, wherein said separation unit includes one or more distillation columns.

4. The method of claim 3, wherein one of said distillation columns is adapted for separation of both methanol and sulfur-containing compounds.

5. The method of claim 1, comprising introducing one or more of said additional sulfur compounds into said alcohol-synthesis reactor to add sulfur to, or reduce sulfur loss from, said sulfided metal catalyst.

6. The method of claim 1, comprising introducing one or more of said additional sulfur compounds into said methanol recycle stream to add sulfur to, or reduce sulfur loss from, said sulfided metal catalyst.

7. The method of any one of the preceding claims, wherein said methanol recycle stream has a sulfur-atom concentration between 50 ppm S and 500 ppm S, more preferred between 100 ppm S and 300 ppm S, and still more preferred of less than 200 ppm S.

## Patentansprüche

1. Verfahren zur Herstellung von Ethanol aus Syngas, wobei das Verfahren umfasst:
(a) Zuführen von Syngas in einen Alkohol-Synthesereaktor, der sulfidierten Metallkatalysator enthält, unter geeigneten Bedingungen zum Umwandeln das Syngases in einen zwischenproduktstrom, der Methanol, Ethanol und eine oder mehrere schwefelhaltige Verbindungen ausgewählt aus Di-tert.-butylpolysulfid (DBPS) und Dimethyldisulfid (DMDS) umfasst;
(b) Leiten mindestens eines Teils dieses Zwischenproduktstroms in eine Trenneinheit, wodurch mindestens ein Teil des Methanols von dem Ethanol getrennt wird, um einen Methanol-Recyclingstrom und einen Ethanol-Produktstrom zu bilden, wobei mindestens ein Teil der schwefelhaltigen Verbindungen in dem Methanol-Recyclingstrom enthalten sind;
(c) Recyceln von etwas oder allem von dem Methanol-Recyclingstrom zu dem Alkohol-Synthesereaktor, um Schwefel zu dem sulfidierten Metallkatalysator zuzuführen oder Schwefelverlust aus dem sulfidierten Metallkatalysator zu verringern, wobei der Methanol-Recyclingstrom eine Schwefelatomkonzentration von mindestens 10 ppm S aufweist; und
(d) gegebenenfalls Einführen von einer oder mehreren zusätzlichen Schwefelverbindungen ausgewählt aus Di-tert.-butylpolysulfid (DBPS) und Dimethyldisulfid (DMDS) in den Alkohol-Synthesereaktor und/oder in den Methanol-Recyclingstrom, um Schwefel zu dem sulfidierten Metallkatalysator zuzuführen oder Schwefelverlust aus dem sulfidierten Metallkatalysator zu verringern.

2. Verfahren nach Anspruch 1, bei dem der sulfidierte Metallkatalysator ein mit Base geförderter Kobalt-Molybdän-Schwefel-Katalysator ist.

3. Verfahren nach Anspruch 1, bei dem die Trenneinheit eine oder mehr Destillationssäulen umfasst.

4. Verfahren nach Anspruch 3, bei dem eine der Destillationssäulen an die Abtrennung von sowohl Methanol als auch schwefelhaltigen Verbindungen angepasst ist.

5. Verfahren nach Anspruch 1, dass die Einführung einer oder mehrerer der zusätzlichen Schwefelverbindungen in den Alkohol-Synthesereaktor umfasst, um Schwefel zu dem sulfidierten Metallkatalysator zuzuführen oder Schwefelverlust aus dem sulfidierten Metallkatalysator zu verringern.

6. Verfahren nach Anspruch 1, dass die Einführung einer oder mehrerer der zusätzlichen Schwefelverbindungen in den Methanol-Recyclingstrom umfasst, um Schwefel zu dem sulfidierten Metallkatalysator zuzuführen oder Schwefelverlust aus dem sulfidierten Metallkatalysator zu verringern.

7. Verfahren nach einem der vorhergehenden Absprüche, bei dem der Methanol-Recyclingstrom eine Schwefelatomkonzentration von 50 ppm S bis 500 ppm S aufweist, bevorzugter 100 ppm S bis 300 ppm S und noch bevorzugter von weniger als 200 ppm S.

## Revendications

1. Procédé de production d'éthanol à partir de gaz de synthèse, ledit procédé comprenant de :
(a) fournir du gaz de synthèse à un réacteur de synthèse d'alcool qui contient un catalyseur métallique sulfuré, dans des conditions adéquates pour convertir ledit gaz de synthèse en un flux intermédiaire contenant du méthanol, de l'éthanol et au moins un composé contenant du soufre choisi parmi le polysulfure de ditertiobutyle (DBPS) et le disulfure de diméthyle (DMDS) ;
(b) envoyer au moins une partie dudit flux intermédiaire à une unité de séparation, grâce à quoi au moins une partie dudit méthanol est séparée dudit éthanol pour former un flux de recyclage de méthanol et un flux de produit éthanol, et où au moins une partie desdits composés contenant du soufre est contenue dans ledit flux de recyclage de méthanol ;
(c) recycler tout ou partie dudit flux de recyclage de méthanol vers ledit réacteur de synthèse d'alcool pour ajouter du soufre ou réduire la perte de soufre dudit catalyseur métallique sulfuré ; où ledit flux de recyclage de méthanol a une concentration en atomes de soufre d'au moins 10 ppm S ; et
(d) introduire facultativement au moins un composé soufré supplémentaire choisi parmi le polysulfure de ditertiobutyle (DBPS) et le disulfure de diméthyle (DMDS) dans ledit réacteur de synthèse d'alcool et/ou dans ledit flux de recyclage de méthanol pour ajouter du soufre ou réduire la perte de soufre dudit catalyseur métallique sulfuré.

2. Procédé selon la revendication 1, dans lequel ledit catalyseur métallique sulfuré est un catalyseur cobalt-molybdène-soufre promu par une base.

3. Procédé selon la revendication 1, dans lequel ladite unité de séparation inclut au moins une colonne de distillation.

4. Procédé selon la revendication 3, dans lequel une desdites colonnes de distillation est adaptée à la séparation à la fois du méthanol et des composés contenant du soufre.

5. Procédé selon la revendication 1, comprenant d'introduire au moins un composé soufré supplémentaire dans ledit réacteur de synthèse d'alcool pour ajouter du soufre ou réduire la perte de soufre dudit catalyseur métallique sulfuré.

6. Procédé selon la revendication 1, comprenant d'introduire au moins un composé soufré supplémentaire dans ledit flux de recyclage de méthanol pour ajouter du soufre ou réduire la perte de soufre dudit catalyseur métallique sulfuré.

7. Procédé selon l'une quelconque des revendications précédentes, dans lequel ledit flux de recyclage de méthanol a une concentration en atomes de soufre entre 50 ppm S et 500 ppm S, plus préférentiellement entre 100 ppm S et 300 ppm S et encore plus préférentiellement inférieure à 200 ppm S.
